(19) Europäisches Patentamt
European Patent Office
Office européen des brevets

(11) EP 2 578 215 A1

(12) **EUROPEAN PATENT APPLICATION**
published in accordance with Art. 153(4) EPC

(43) Date of publication:
**10.04.2013 Bulletin 2013/15**

(21) Application number: **11786163.3**

(22) Date of filing: **13.05.2011**

(51) Int Cl.:
***A61K 31/194*** *(2006.01)* ***A61K 9/08*** *(2006.01)*
***A61P 9/00*** *(2006.01)*

(86) International application number:
**PCT/ES2011/070344**

(87) International publication number:
**WO 2011/148014 (01.12.2011 Gazette 2011/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.05.2010 ES 201030786**

(71) Applicants:
- **Universidade De Santiago De Compostela**
  **15782 Santiago de Compostela (ES)**
- **Servizo Galego De Saúde**
  **15782 Santiago de Compostela (La Coruña) (ES)**
- **Fundación Para la Investigación, Desarrollo E Innovación del Complejo Hospitalario Universitario de Santiago (IDICHUS)**
  **15782 Santiago de Compostela (La Coruña) (ES)**

(72) Inventors:
- **CASTILLO SÁNCHEZ, José**
  **E-15782 Santiago de Compostela (La Coruña) (ES)**
- **SOBRINO MOREIRAS, Tomás**
  **E-15782 Santiago de Compostela (La Coruña) (ES)**
- **RAMOS CABRER, Pedro**
  **E-15782 Santiago de Compostela (La Coruña) (ES)**
- **CAMPOS PEREZ, Francisco**
  **E-15782 Santiago de Compostela (La Coruña) (ES)**
- **AGULLA FREIRE, Jesús**
  **E-15782 Santiago de Compostela (La Coruña) (ES)**
- **ARGIBAY GONZÁLEZ, Bárbara**
  **E-15782 Santiago de Compostela (La Coruña) (ES)**

(74) Representative: **ABG Patentes, S.L.**
**Avenida de Burgos, 16D**
**Edificio Euromor**
**28036 Madrid (ES)**

# (54) USE OF OXALOACETATE IN THE TREATMENT OF ISCHAEMIA

(57) The invention relates to the use of oxaloacetate in the treatment of ischaemia. The invention provides quantities that are therapeutically effective for the treatment of disorders associated with high levels of glutamate in brain tissue, such as, for example, cerebral ischaemia. In addition, the compositions of the invention are suitable for bolus-type delivery.

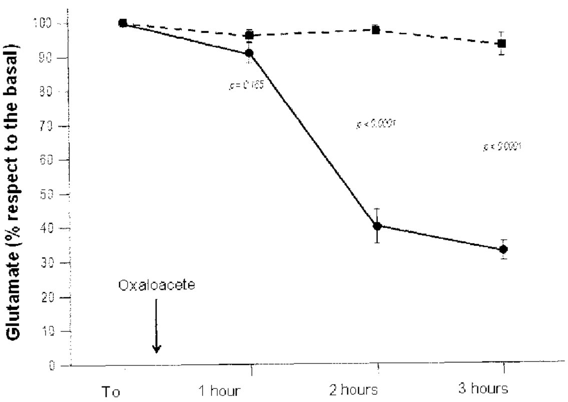


Figure 1a



Printed by Jouve, 75001 PARIS (FR)

(Cont. next page)

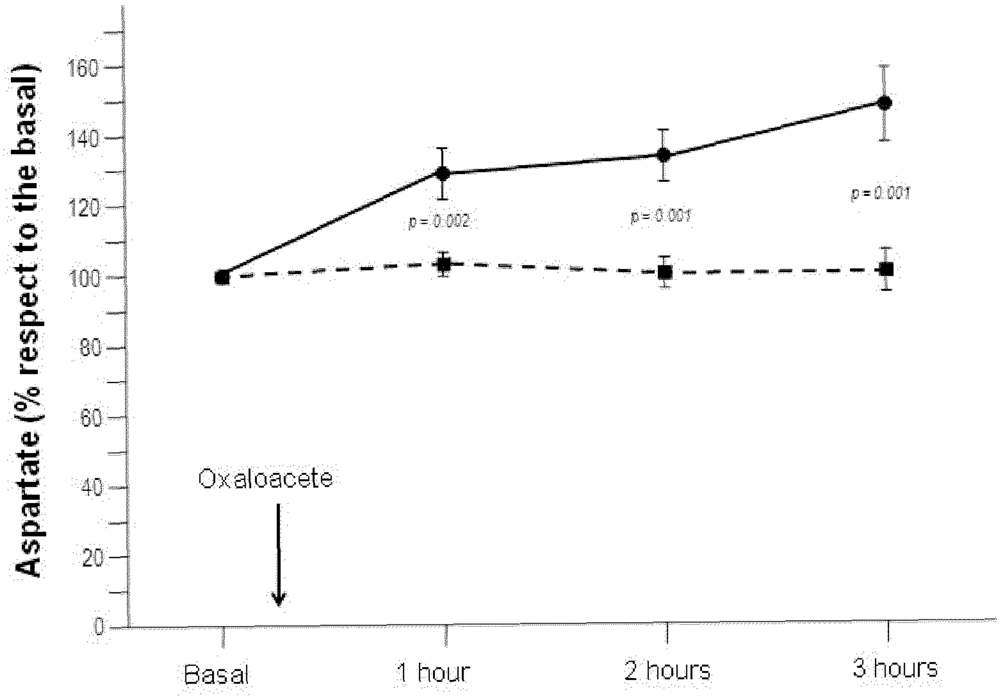
Aspartate (% respect to the basal)
160
140
120
100
80
60
40
20
0
p = 0.002
p = 0.001
p = 0.001
Oxaloacete
Basal
1 hour
2 hours
3 hours

**Figure 1b**

## Description

### Field of the invention

This invention relates to the field of medicine for the treatment of disorders associated with high glutamate concentrations in brain tissue, such as cerebral ischaemia.

### Background

Stroke is one of the leading causes of death in the developed world and can also provoke severe disability in terms of both the degree of dependence of survivors and the way in which it affects many working age people. Fewer than half of those patients who suffer stroke are able to resume a normal life (the prevalence of incomplete recovery is 460/1000 survivors). Such people subsequently need to be cared for by their family, thereby affecting the quality of life of both patients and their caregivers, or by public or private institutions, which requires a substantial economic investment.

Stroke has two main causes: the thrombotic obstruction of a blood vessel (ischaemic stroke, which accounts for 80% of cases) and haemorrhage (haemorrhagic stroke, due to a burst blood vessel, which accounts for the remaining 20%). Although the symptoms of stroke (loss of strength on one side of the body, difficulty speaking and understanding, partial loss of vision and vertigo, amongst others) appear suddenly, some time must pass before neuronal death-related brain damage occurs. This time period is known as the "therapeutic window" during which neurologists must attempt to prevent neuronal death in the region of the brain affected by the stroke or haemorrhage.

Despite the high incidence of stroke, its treatment is currently limited to unblocking the obstructed vessels using fibrinolytic agents such as t-PA (tissue plasminogen activator), this being the only effective treatment proven to date. However, as such treatment is often associated with severe side-effects, its application is restricted to around 1% of patients. As a result, only a small percentage of patients can benefit from this therapy (Adams, H.P., Jr., et al., Classification of subtype of acute ischemic stroke. Definitions for use in a multicenter clinical trial. TOAST. Trial of Org 10172 in Acute Stroke Treatment. Stroke, 1993. 24(1): p. 35-41).

The limited number of therapeutic options available to treat this disease means that new therapies which could be applied in a larger number of patients must be developed.

In this regard, many of the large number of neuroprotective agents discovered during experimental research failed during clinical trials due to the narrow therapeutic window available (Castellanos, M., T. Sobrino, and J. Castillo, Evolving paradigms for neuroprotection: molecular identification of ischemic penumbra. Cerebrovasc Dis, 2006. 21 Suppl 2: p. 71-9). A further important reason for this failure is the poor quality and suitability of the tests for neuroprotective agents against stroke in animals (Philip, M., et al., Methodological quality of animal studies of neuroprotective agents currently in phase II/III acute ischemic stroke trials. Stroke, 2009. 40(2): p. 577-81).

It is well known that glutamate is the initial trigger for neuronal death and damage in the initial acute phase of stroke (Lipton, P., Ischemic cell death in brain neurons. Physiol Rev, 1999. 79(4): p. 1431-568). Thus, stroke patients present higher levels of glutamate in blood, and prolonged high glutamate levels in both blood and cerebrospinal fluid (CSF) have been linked to an increased neurological deficit in stroke patients (Castillo, J., et al., Neuroexcitatory amino acids and their relation to infarct size and neurological deficit in ischemic stroke. Stroke, 1996. 27(6): p. 1060-5), which could be due to increased cell death resulting from the excitotoxic action of glutamate. Indeed, blood glutamate concentrations of more than 200 $\mu$M are an important predictor of the progression of neuronal damage (Castillo, J., A. Davalos, and M. Noya, Progression of ischaemic stroke and excitotoxic amino acids. Lancet, 1997. 349(9045): p. 79-83).

The fact that glutamate plays a key role in the cascade of molecular events that take place after an ischaemic process and is also closely linked to the resulting neurological deterioration makes it an obvious target in the search for new neuroprotective agents against ischaemic stroke.

Thus, the prior art includes studies concerning the neuroprotective activity of oxaloacetate (Nagy, D., et al., Oxaloacetate decreases the infarct size and attenuates the reduction in evoked responses after photothrombotic focal ischaemia in the rat cortex. Cell Mol Neurobiol, 2009. 29(6-7): p. 827-35). In these examples oxaloacetate is administered intravenously by continuous infusion over a specified time period.

However, the fact that glutamate-induced damage occurs in the first few hours post-stroke, and therefore that any intervention to control glutamate levels should be initiated as soon as possible to prevent its effects, and in a fast and effective manner, must be taken into account. This highlights the importance of the development of simple strategies that can be applied by specialised technical personnel during transport of the patient to hospital in the ambulance.

### Description of the invention

This invention describes a simple method for the treatment or prevention of disorders associated with high glutamate concentrations in brain tissue in the first few hours after they occur.

This method is based on the use of a therapeutically effective amount of oxaloacetate as an activator of the enzyme glutamate oxaloacetate transaminase (GOT). This activation results in a reduction in blood glutamate levels and a reduction in glutamate levels in brain tissue, thereby inducing a neuroprotective effect.

Although the prior art describes the neuroprotective activity of oxaloacetate, the amounts used are not effective when administered as a bolus in animals according to the STAIR guidelines for the preclinical evaluation of stroke therapy (Philip, M., et al., Methodological quality of animal studies of neuroprotective agents currently in phase II/III acute ischemic stroke trials. Stroke, 2009. 40(2): p. 577-81). Thus, surprisingly, and in contrast to the prior art (Nagy, D., et al., Oxaloacetate decreases the infarct size and attenuates the reduction in evoked responses after photothrombotic focal ischaemia in the rat cortex. Cell Mol Neurobiol, 2009. 29(6-7): p. 827-35), this invention demonstrates that 1.2 mg/100 g is not a therapeutically effective amount, as established previously, to either produce a beneficial effect, or induce a neuroprotective effect after a cerebral ischaemic process, or reduce glutamate levels in blood or brain tissue.

An advantage of this invention is that it provides therapeutically effective amounts of oxaloacetate for the treatment of disorders associated with high glutamate concentrations in brain tissue. A further advantage is the use of therapeutically effective amounts of oxaloacetate to prepare a medicinal product for administration via bolus.

The administration via bolus of a medicinal product for the treatment of disorders associated with high glutamate concentrations in brain tissue implies a higher probability of success than in the case of continuous intravenous infusion as beneficial effects will only be produced if said product is administered within the "therapeutic window". This approach solves the problems in the prior art.

Thus, this invention relates to the use of oxaloacetate or its derivatives for the manufacturing of a medicinal product for administration via bolus in a therapeutically effective amount for the treatment or prevention of a pathology associated to high concentrations of glutamate in brain tissue.

Likewise, this invention provides ranges for a therapeutically effective amount of oxaloacetate or its derivatives based on extrapolation of the data obtained in animal models, according to the STAIR guidelines for the preclinical evaluation of stroke therapy, as described in "Dose translation from animal to human studies revisited", The FASEB Journal, 2007, 22, 659-661. It should also be noted that this invention describes, for the first time, the results of an oxaloacetate activity study in these ischaemia models, which better represent ischaemic stroke disease in humans (Carmichael, S.T., Rodent models of focal stroke: size, mechanism, and purpose. NeuroRx, 2005. 2(3): p. 396-409).

The invention also describes a pharmaceutical composition, a kit and a treatment or prevention method.

**Detailed description of the invention**

In this invention, the term "administration via bolus" refers to the intravenous (IV) administration of a medicinal product in a fast and controlled manner, for example between 1 and 10 seconds, preferably between 1 and 5 seconds and more preferably between 2 and 3 seconds This contrasts with infusion-type administration, which occurs over a longer time period, for example between 30 minutes and 8 hours, or drip-type administration.

In this invention, "high glutamate concentrations in brain tissue" are determined by in vitro analysis of the glutamate concentration in blood, and the glutamate concentration in brain tissue is considered to be high when the glutamate concentration in blood exceeds 200 $\mu$M (Castillo, J., A. Davalos, and M. Noya, Progression of ischaemic stroke and excitotoxic amino acids. Lancet, 1997. 349(9045): p. 79-83).

Similarly, the term "oxaloacetate" as used in this invention refers to the deprotonated derivative of oxaloacetic acid with the formula $[C_4H_2O_5]^{2-}$. The term "its derivatives" refers to the partially (conjugate base) or totally protonated form (oxaloacetic acid), its pharmaceutically acceptable salts, and its esters, such as diethyl oxaloacetate, and salts such as the disodium salt of diethyl oxaloacetate. Oxaloacetate is commercially available as a salt, for example the sodium salt. As a metabolite it is involved in various metabolic pathways such as the Krebs cycle or C-4 photosynthesis.

The enzyme glutamate oxaloacetate transaminase (GOT) is a liver enzyme which, in the presence of oxaloacetate, metabolises glutamate to form aspartate and $\alpha$-ketoglutarate as degradation products according to the following scheme:

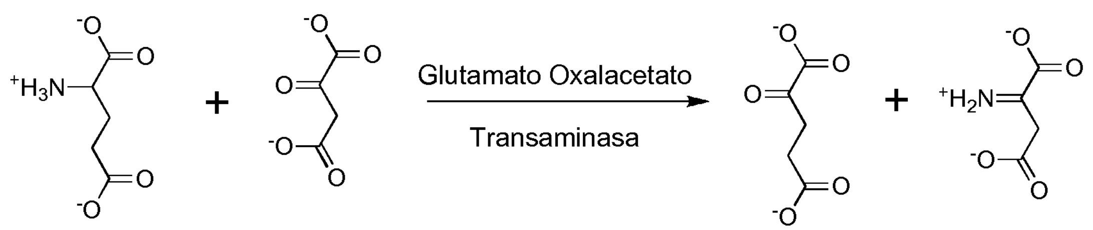


Glutamato Oxalacetato Aspartato a-Cetoglutarato

The use of a therapeutically effective amount of oxaloacetate or its derivatives as a GOT activator, as described in this invention, speeds up the aforementioned reaction, thus decreasing the glutamate concentration in blood. This results in a more pronounced glutamate concentration gradient between the brain and the blood, thereby promoting the outflow of extracellular glutamate from the cerebral parenchyma.

The rapid outflow of glutamate released by cells that have lost their energy capabilities, or dead cells, from the cerebral parenchyma results in less damage to healthy tissue and therefore a reduction in the neurological deficit that occurs in those patients with disorders associated with high glutamate concentrations in brain tissue.

According to a particular embodiment, a pathology associated to high concentrations of glutamate is preferably selected from the group comprising cerebral ischaemia, trauma brain injury, glaucoma and amyotrophic lateral sclerosis, more preferably cerebral ischaemia.

Likewise, in a particular embodiment, this invention concerns the use of oxaloacetate or its derivatives for manufacturing a medicinal product for administration via bolus in a therapeutically effective amount for the treatment or prevention of a pathology associated to high concentrations of glutamate in brain tissue selected from the group comprising cerebral ischaemia, trauma brain injury, glaucoma and amyotrophic lateral sclerosis.

In a more particular embodiment, this invention concerns the use of oxaloacetate or its derivatives for the manufacturing of a medicinal product for administration via bolus in a therapeutically effective amount for the treatment or prevention of cerebral ischaemia.

In a further embodiment, this invention concerns the use of oxaloacetate or its derivatives for the manufacturing of a medicinal product for administration via bolus in a therapeutically effective amount to decrease glutamate levels in blood. In a further embodiment, this invention concerns the use of oxaloacetate or its derivatives for the manufacturing of a medicinal product for administration via bolus in a therapeutically effective amount to decrease glutamate levels in brain tissue. In a further embodiment, this invention concerns the use of oxaloacetate or its derivatives for the manufacturing of a medicinal product for administration via bolus in a therapeutically effective amount to induce a neuroprotective effect.

As used in this invention, the term "therapeutically effective amount" refers to the specific dose of oxaloacetate or its derivatives that produces a beneficial effect in humans or animals, in other words by treating or preventing brain tissue pathologies associated to high levels of glutamate, preferably a pathology selected from the group comprising cerebral ischaemia, brain trauma injury, glaucoma and amyotrophic lateral sclerosis, more preferably cerebral ischaemia, whilst minimising side-effects. The therapeutically effective amount is preferably administered in the time period between occurrence of the brain tissue disorder associated with high concentrations of glutamate for the treatment and prevention of brain tissue pathologies, the so-called "therapeutic window". A preferred time period is less than 3 hours from an increase of glutamate levels in brain tissue. A more preferred time period is less than 2 hours, and an even more preferred time period is 90 minutes or less.

The therapeutically effective amount of oxaloacetate or its derivatives preferred for this invention is greater than 2.4 mg/Kg of patient weight, preferably between 4 mg/Kg of patient weight and 8 mg/Kg of patient weight, and more preferably between 5 mg/Kg of patient weight and 6 mg/Kg of patient weight.

In a further embodiment, the present invention relates to a pharmaceutical composition comprising oxaloacetate or its derivatives in a therapeutically effective amount for administration via bolus. This composition may be used to treat or prevent a pathology associated to high concentrations of glutamate in brain tissue, preferably a pathology associated to high concentrations of glutamate in brain tissue selected from the group comprising cerebral ischaemia, brain trauma injury, glaucoma and amyotrophic lateral sclerosis, more preferably to treat or prevent cerebral ischaemia.

The pharmaceutical compositions of interest for this invention can be prepared using procedures known in the art. For example, the compounds can be formulated with standard excipients, diluents and vehicles, and can be presented in solution or suspension for intravenous administration. The pharmaceutical composition preferably has a pH of between 7.2 and 7.4.

In a particular embodiment of the invention, the pharmaceutical composition defined above comprises a therapeutically effective amount of oxaloacetate or its derivatives higher than 2.4 mg/Kg of patient weight, preferably between 4 mg/Kg of patient weight and 8 mg/Kg of patient weight, and more preferably between 5 mg/Kg of patient weight and 6 mg/Kg of patient weight.

In a further particular embodiment, the pharmaceutical composition defined above comprises a therapeutically effective amount of oxaloacetate or its derivatives in a solution of a total volume between 1 mL and 5 mL, preferably a total volume between 2 mL and 4 mL, and more preferably 2.5 mL. The volumes of the pharmaceutical composition make it suitable for administration via bolus.

Thus, in a particular embodiment, the pharmaceutical composition of this invention is a solution comprising a therapeutically effective amount of oxaloacetate or its derivatives at a concentration higher than 35 mg/mL, preferably between 50 and 500 mg/mL, more preferably between 110 and 225 mg/mL, more preferably between 140 and 180 mg/mL and even more preferably between 160 and 170 mg/mL. For example, a pharmaceutical composition with a total volume of 2.5 mL is suitable for an amount of oxaloacetate or its derivatives of approximately 6 mg/Kg. In this case, and

supposing an average patient weight of 70 Kg, the concentration of the composition is between 160 and 170 mg/Kg (approximately 168 mg/Kg).

This invention provides data which demonstrate that the administration via bolus of oxaloacetate or its derivatives in a therapeutically effective amount leads to a decrease in blood glutamate levels. As a result, the level of glutamate in the cerebral parenchyma also decreases. The presence of high glutamate levels in the blood decreases the blood-brain gradient, thereby reducing the ease with which excess glutamate can be removed from the brain. In contrast, low levels of glutamate in the blood enhance the drainage of cerebral glutamate into the bloodstream. This mechanism of action allows the excitotoxicity produced by high glutamate concentrations, and therefore the damage caused by such concentrations in brain tissue, to be reduced. Especially cerebral ischaemia. Thus, this oxaloacetate-based treatment that targets liver enzyme (GOT) activity is shown to be effective at reducing plasma glutamate levels and glutamate levels in brain tissue, thereby reducing ischaemic brain damage and inducing a neuroprotective effect.

In a further embodiment, this invention relates to a kit comprising a pharmaceutical composition as defined above for the treatment or prevention of a pathology associated to high concentrations of glutamate in brain tissue.

The invention also relates to a method for prevention or treatment of a pathology associated to high concentrations of glutamate in brain tissue which comprises administration via bolus of a therapeutically effective amount of oxaloacetate or its derivatives, preferably a pathology associated to high concentrations of glutamate in brain tissue selected from the group comprising cerebral ischaemia, brain trauma injury, glaucoma and amyotrophic lateral sclerosis, more preferably to treat or prevent cerebral ischaemia.

In a particular embodiment, the therapeutically effective amount of oxaloacetate or its derivatives is preferably that of interest for the invention described above.

In a further particular embodiment, this therapeutically effective amount of oxaloacetate or its derivatives is found in solution at a concentration higher than 35 mg/mL, preferably between 50 and 500 mg/mL, more preferably between 110 and 225 mg/mL, more preferably between 140 and 180 mg/mL and even more preferably between 160 and 170 mg/mL.

In another particular embodiment, the solution has a total volume of between 1 mL and 5 mL, preferably a total volume of between 2 mL and 4 mL, and more preferably 2.5 mL.

In a further particular embodiment, the prevention or treatment method described above is applied within a time period of less than 3 hours from the onset of the increase of glutamate levels in brain tissue. A more preferred time period is less than 2 hours, and an even more preferred time period is 90 minutes or less.

In another embodiment, this invention also relates to a method for prevention or treatment of a pathology associated to high concentrations of glutamate in brain tissue comprising administration via bolus of a pharmaceutical composition defined above.

**Description of the figures**

Figure 1. Dose-response effect of oxaloacetate on blood glutamate (Figure 1a) and aspartate (Figure 1b) levels: oxaloacetate dose of 1.5 mg/100 g (IV) (n=3) ■; oxaloacetate dose of 3.5 mg/100 g (IV) (n=4) ●. The glutamate and aspartate values are shown as ±SEM and expressed as a percentage of basal levels. The results were evaluated statistically using Student's t-test.

Figure 2. Evolution of blood glutamate levels, expressed as concentration (micromolar) versus time, in control animals ♦ and in an animal model of ischaemia (MCAO treated) ✦. Glutamate levels are shown as ±SEM. The results were evaluated statistically using Student's t-test.

Figure 3. Evolution of infarct (Figure 3a) and oedema volume (Figure 3b) versus time for the control ♦ and treated groups ✦ (treated 90 min after temporary occlusion of the middle cerebral artery) (MCAO). The infarct and oedema volumes are shown as ±SEM. The results were evaluated statistically using Student's t-test.

Figure 4. Shows the results of a somatosensory test for the control and treated groups.

The following examples are provided for illustrative purposes only and should not be considered to limit this invention's application.

**Examples**

The oxaloacetate used (Sigma-Aldrich, Ref, 171263, 95%) was dissolved in saline solution to a concentration of 10 mg/mL and the pH adjusted to 7.4. As the average weight of the experimental animals was 325 g and the dose administered was 3.5 mg/100 g body weight, each animal received an average volume of 1.2 mL of 10 mg/mL oxaloacetate.

The study protocols for male Sprague-Dawley (SD) rats (Charles River Laboratories; France) weighing between 300 and 350 g were approved by the Animal Use and Care Committee at the Hospital de Santiago de Compostela.

Anaesthesia was induced by inhalation of 5% sevoflurane in a mixture of nitrous oxide and oxygen (70/30). A rectal temperature of 37.5 °C was maintained. Glucose levels (180-220 mg/dL) were analysed prior to the intervention.

The amounts of oxaloacetate administered to rats were extrapolated to a human dose as described in "Dose translation from animal to human studies revisited", The FASEB Journal, 2007, 22, 659-661, using the equation:

$$\text{Dose in humans (mg/kg)} = \text{Dose in animal (mg/kg)} * (\text{km animal / km human})$$

and a value of 6 for the km in animal and 37 for the km in humans.

The examples were designed to ensure that oxaloacetate was administered within a broad therapeutic window according to the STAIR guidelines for the preclinical evaluation of stroke therapy (Philip, M., et al., Methodological quality of animal studies of neuroprotective agents currently in phase II/III acute ischemic stroke trials. Stroke, 2009. 40(2): p. 577-81).

Middle cerebral artery occlusion (MCAO) was used as a model of cerebral ischaemia, as described in the literature (Longa, E.Z., et al., Reversible middle cerebral artery occlusion without craniectomy in rats. Stroke, 1989. 20 (1): p. 84-91). This model is considered to best mimic cerebral ischaemia in humans and has been used in numerous rat studies (Carmichael, S.T., Rodent models of focal stroke: size, mechanism, and purpose. NeuroRx, 2005. 2(3): p. 396-409).

Aspartate and glutamate levels in blood were analysed according to the method described in the following reference (White, J.A., R.J. Hart, and J.C. Fry, An evaluation of the Waters Pico-Tag system for the amino-acid analysis of food materials. J Automat Chem, 1986. 8(4): p. 170-7).

The infarct and oedema volumes were determined by magnetic resonance imaging. These experiments were performed using a 9.4 T machine (Bruker BioSpin, Germany) with a horizontal chamber 20 cm in diameter and gradients of 440 mT and 250 ms. All images were processed using the ImageJ software (Rasband, W.S., ImageJ, NIH, http: //rsb.info.nih.gov/ij).

Somatosensory studies were performed as described in (Reglodi, D., A. Tamas, and I. Lengvari, Examination of sensorimotor performance following middle cerebral artery occlusion in rats. Brain Res Bull, 2003. 59(6): p. 459-66).

**Example 1.** Effect of the administration of oxaloacetate on serum glutamate levels.

An oxaloacetate dose of 1.5 mg/100 g (IV) was administered to healthy animals and no changes in blood glutamate levels were observed after 3 hours (Figure 1a). Likewise, no changes in aspartate levels were observed (Figure 1b).

The administration of a higher dose of oxaloacetate (3.5 mg/100 g IV) to healthy animals resulted in a 60% decrease in serum glutamate levels with respect to basal levels two hours post-administration. This effect was maintained up to three hours post-administration (Figure 1a). A concomitant increase in aspartate levels at two and three hours after treatment with oxaloacetate was observed (Figure 1b). This increase in aspartate levels confirms that glutamate is being metabolised by the enzyme GOT as a result of oxaloacetate treatment.

The oxaloacetate and aspartate levels in blood were established in a control group treated with saline.

These results demonstrate that the administration via bolus of 1.2 mg/100 g (IV) of oxaloacetate (Nagy, D., et al., Oxaloacetate decreases the infarct size and attenuates the reduction in evoked responses after photothrombotic focal ischaemia in the rat cortex. Cell Mol Neurobiol, 2009. 29(6-7): p. 827-35) has no effect on glutamate levels and is therefore not an effective dose according to this invention.

**Example 2.** The effect of oxaloacetate administration on blood glutamate levels in an animal model of focal ischaemia.

Two groups were designed: an MCAO group treated with saline (control group) and an MCAO group treated with 3.5 mg/100 g oxaloacetate IV 90 minutes post-occlusion. The blood glutamate levels and infarct and oedema volumes were determined in both groups. A somatosensory test was performed on day 7.

The control group showed a 40% increase in blood glutamate levels at 4 hours post-occlusion. Glutamate returned to normal levels at 24 hours post-occlusion (Figure 2).

The glutamate levels for the treatment group dropped 50% below basal levels at 4 hours post-occlusion. They returned to basal at 24 hours post-occlusion (Figure 2).

The infarct volume was approximately 40% smaller in the treatment group than in the control group at 24 hours post-ischaemia. At 7 days this volume decreased by 80% in the treatment group and 20% in the control group (Figure 3a).

The treatment group showed no increase in oedema volume, whereas a marked increase was observed for the control group in comparison with the treatment group on day 3 (Figure 3b).

In light of the data obtained in this study, and extrapolating to the human dose using the equation mentioned above, the dose of oxaloacetate for human patients is approximately 6 mg/kg. Considering an average weight of 70 kg, a suitable amount of oxaloacetate for administration to a human patient is approximately 420 mg. A volume (bolus) of 2.5 mL is suitable for administration to a human patient. Thus, a pharmaceutical composition with a concentration of 420 mg oxaloacetate/2.5 mL, in other words 168 mg/mL, can be prepared, performing the dilution in saline solution with a pH of 7.4 and 0.9% NaCl.

**Example 3.** Somatosensory test.

The somatosensory test was performed on a scale of 0 to 4 such that those animals which presented greater somatosensory alterations were assigned a score of 4. The test performed showed that the treatment group had a higher score than the control group (Figure 4).

## Claims

1. Use of oxalacetate or its derivatives for the manufacturing of a medicament for its administration via bolus in a therapeutically effective amount for the treatment or prevention of a pathology associated to high concentrations of glutamate in the brain tissue.

2. Use according to claim 1, wherein the pathology associated to high concentrations of glutamate in the brain tissue is selected from the group consisting of cerebral ischemia, traumatic brain injury, glaucoma and amyotrophic lateral sclerosis.

3. Use according to claims 1 and 2, wherein the pathology is cerebral ischemia.

4. Use of oxalacetate or its derivatives for the manufacturing of a medicament for its administration via bolus in a therapeutically effective amount for reducing the levels of glutamate in blood.

5. Use of oxalacetate or its derivatives for the manufacturing of a medicament for its administration via bolus in a therapeutically effective amount to decrease the levels of glutamate in the brain tissue.

6. Use of oxalacetate or its derivatives for the manufacturing of a medicament for its administration via bolus in a therapeutically effective amount to induce neuroprotective effect.

7. Use according to any of previous claims, wherein the therapeutically effective amount of oxalacetate or its derivatives is higher than 2.4 mg/Kg of patient weight.

8. Use according to any of previous claims, wherein the therapeutically effective amount of oxalacetate or its derivatives is between 4 mg/Kg and 8 mg/Kg.

9. Use according to any of previous claims, wherein the therapeutically effective amount of oxalacetate or its derivatives is between 5 mg/Kg and 6 mg/Kg.

10. Pharmaceutical composition comprising oxalacetate or its derivatives in a therapeutically effective amount for administration via bolus for the treatment or prevention of a pathology associated to high concentrations of glutamate in the brain tissue.

11. Pharmaceutical composition according to claim 10, wherein the pathology associated to high concentrations of glutamate in the brain tissue is selected from the group consisting of cerebral ischemia, traumatic brain injury, glaucoma and amyotrophic lateral sclerosis.

12. Pharmaceutical composition according to claims 10 and 11, wherein the pathology associated to high concentrations of glutamate in the brain tissue is cerebral ischemia.

13. Pharmaceutical composition according to claims 10 to 12, wherein the therapeutically effective amount of oxalacetate

or its derivatives is higher than 2.4 mg/Kg of patient weight.

**14.** Pharmaceutical composition according to claims 10 to 12, wherein the therapeutically effective amount of oxalacetate or its derivatives is between 4 mg/Kg and 8 mg/Kg.

**15.** Pharmaceutical composition according to claims 10 to 12, wherein the therapeutically effective amount of oxalacetate or its derivatives is between 5 mg/Kg and 6 mg/Kg.

**16.** Pharmaceutical composition according to claims 10 to 15, wherein the therapeutically effective amount of oxalacetate or its derivatives is in a solution of a total volume between 1 mL and 5 mL.

**17.** Pharmaceutical composition according to claims 10 to 15, wherein the therapeutically effective amount of oxalacetate or its derivatives is in a solution of a total volume between 2 mL and 4 mL.

**18.** Pharmaceutical composition according to claims 10 to 15, wherein the therapeutically effective amount of oxalacetate or its derivatives is in a solution of a total volume of 2.5 mL.

**19.** Kit comprising a pharmaceutical composition as defined in any of claims 10 to 18 for the treatment or prevention of a pathology associated to high concentrations of glutamate in the brain tissue.

**20.** Method for the treatment or prevention of a pathology associated to high concentrations of glutamate in the brain tissue which comprises the administration via bolus of oxalacetate or its derivatives in a therapeutically effective amount.

**21.** Method for the treatment or prevention according to claim 20, wherein the pathology associated to high concentrations of glutamate in the brain tissue is selected from the group consisting of cerebral ischemia, traumatic brain injury, glaucoma and amyotrophic lateral sclerosis.

**22.** Method for the treatment or prevention according to claims 20 and 21, wherein the pathology associated to high concentrations of glutamate in the brain tissue is cerebral ischemia.

**23.** Method for the treatment or prevention according to claims 20 and 22, wherein the therapeutically effective amount of oxalacetate or its derivatives is higher than 2.4 mg/Kg of patient weight.

**24.** Method for the treatment or prevention according to claims 20 and 22, wherein the therapeutically effective amount of oxalacetate or its derivatives is between 4 mg/Kg and 8 mg/Kg.

**25.** Method for the treatment or prevention according to claims 20 and 22, wherein the therapeutically effective amount of oxalacetate or its derivatives is between 5 mg/Kg and 6 mg/Kg.

**26.** Method for the treatment or prevention according to claims 20 and 25, wherein the therapeutically effective amount of oxalacetate or its derivatives is in a solution of a total volume between 1 mL and 5 mL.

**27.** Method for the treatment or prevention according to claims 20 and 25, wherein the therapeutically effective amount of oxalacetate or its derivatives is in a solution of a total volume between 2 mL and 4 mL.

**28.** Method for the treatment or prevention according to claims 20 and 25, wherein the therapeutically effective amount of oxalacetate or its derivatives is in a solution of a total volume of 2.5 mL.

**29.** Method for the treatment or prevention according to claims 20 and 28, which is applied into a time period less than 3 hours from the increasing of the levels of glutamate in the brain tissue.

**30.** Method for the treatment or prevention according to claims 20 and 28, which is applied into a time period less than 2 hours from the increasing of the levels of glutamate in the brain tissue.

**31.** Method for the treatment or prevention according to claims 20 and 28, which is applied into a time period less than 90 minutes from the increasing of the levels of glutamate in the brain tissue.

**32.** Method for the treatment or prevention of a pathology associated to high concentrations of glutamate in the brain tissue which comprises the administration via bolus of a pharmaceutical composition as defined in claims 10 to 18.

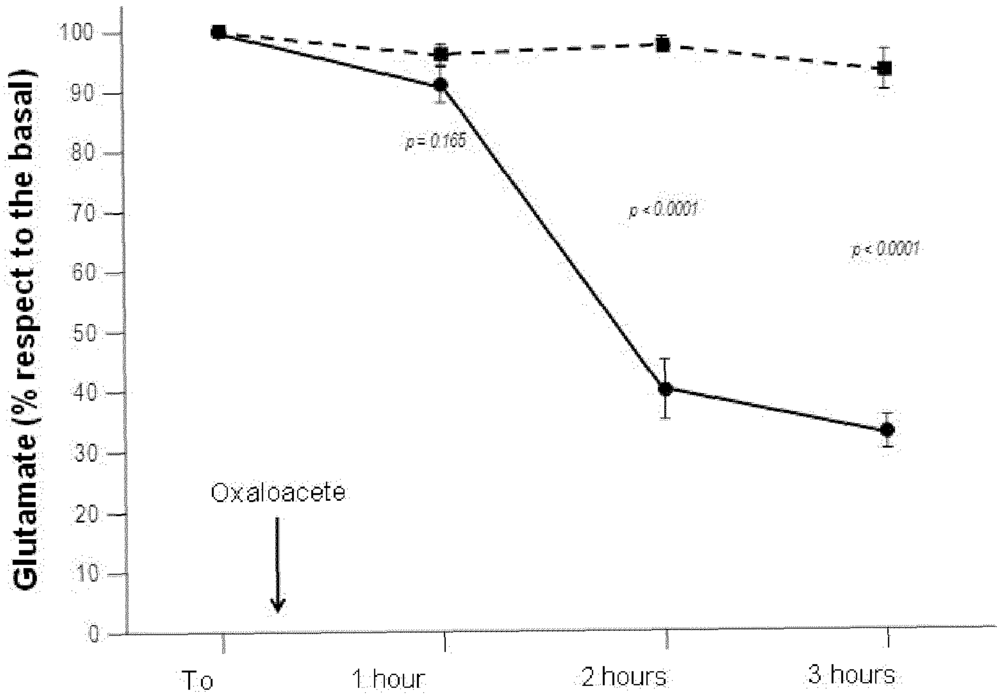


**Figure 1a**

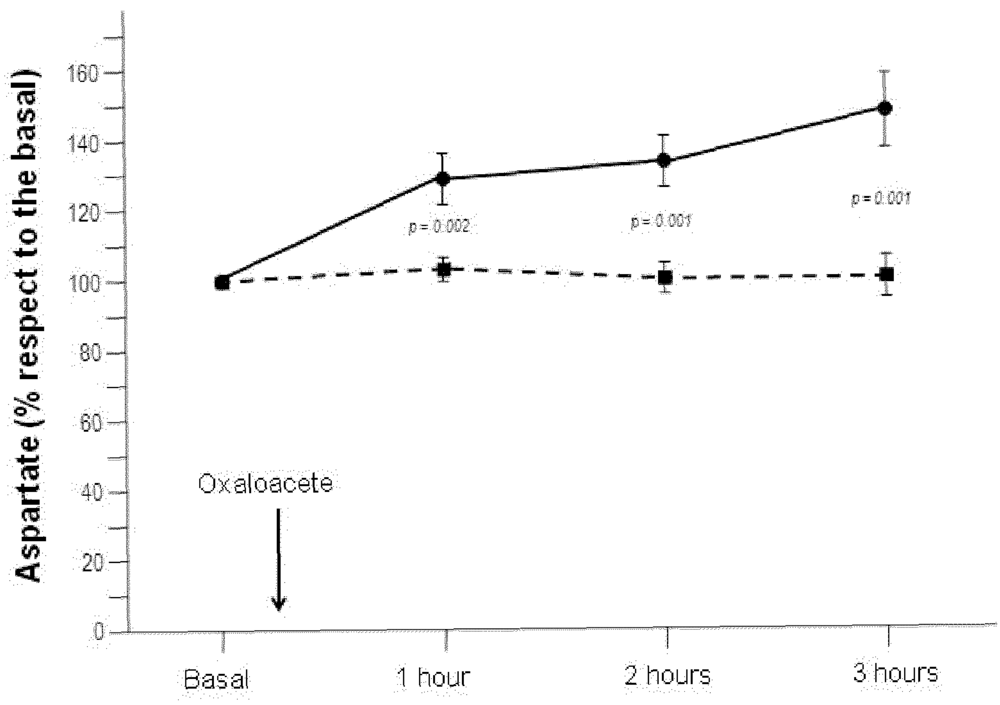


**Figure 1b**

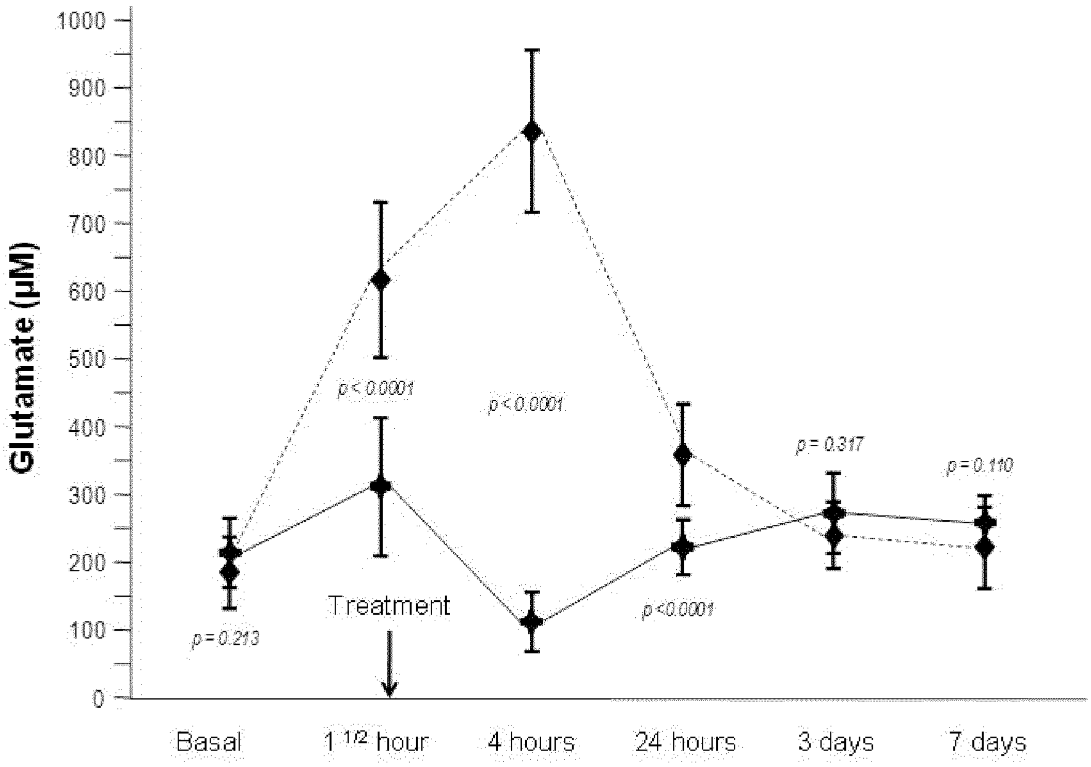
Glutamate (µM)
1000
900
800
700
600
500
400
300
200
100
0
p < 0.0001
p < 0.0001
p = 0.317
p = 0.110
p = 0.213
Treatment
p <0.0001
Basal
1 1/2 hour
4 hours
24 hours
3 days
7 days

**Figure 2**

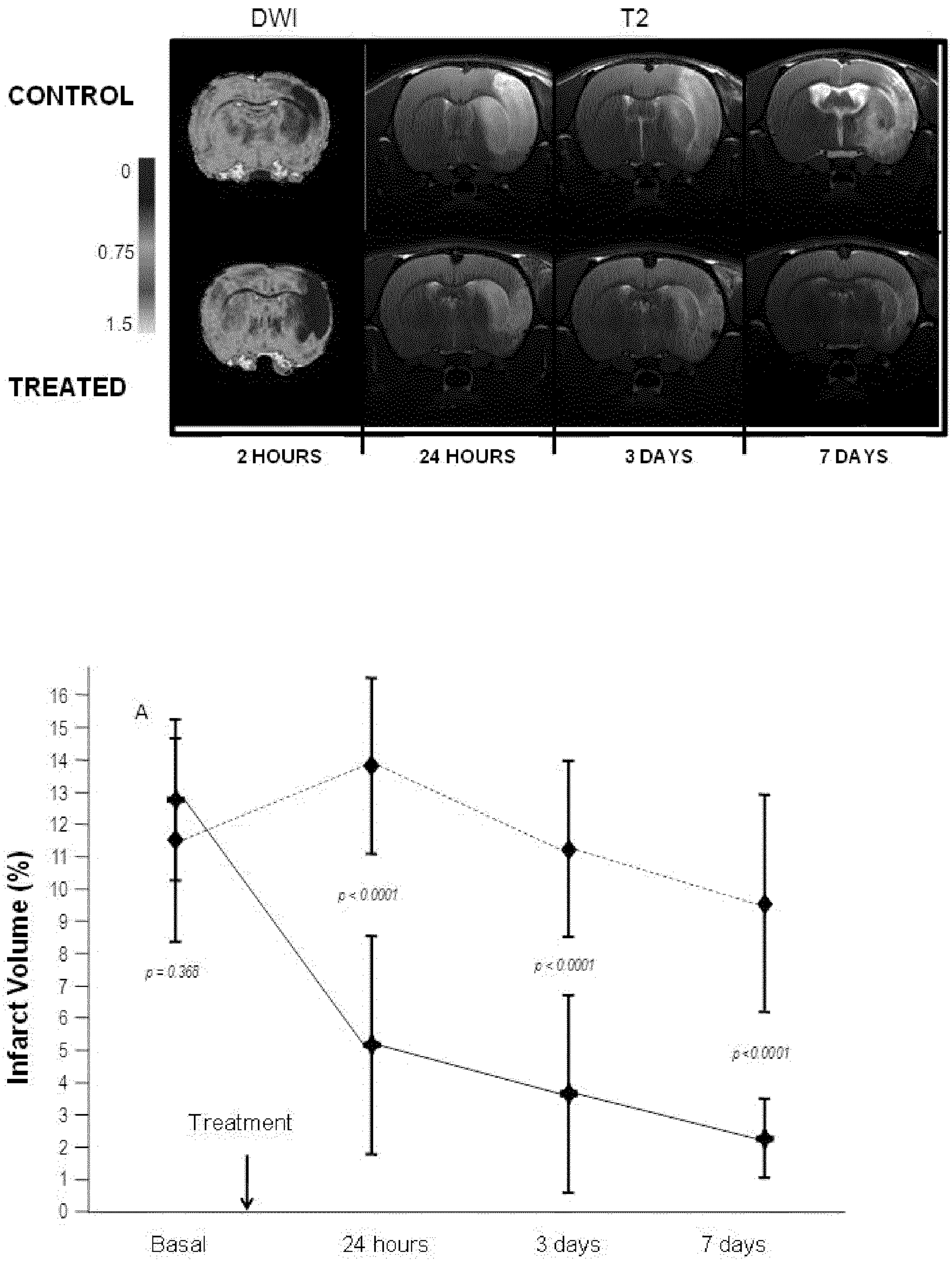
DWI
T2
CONTROL
0
0.75
1.5
TREATED
2 HOURS
24 HOURS
3 DAYS
7 DAYS
A
Infarct Volume (%)
16
15
14
13
12
11
10
9
8
7
6
5
4
3
2
1
0
p = 0.366
p < 0.0001
p < 0.0001
p < 0.0001
Treatment
Basal
24 hours
3 days
7 days

**Figure 3a**

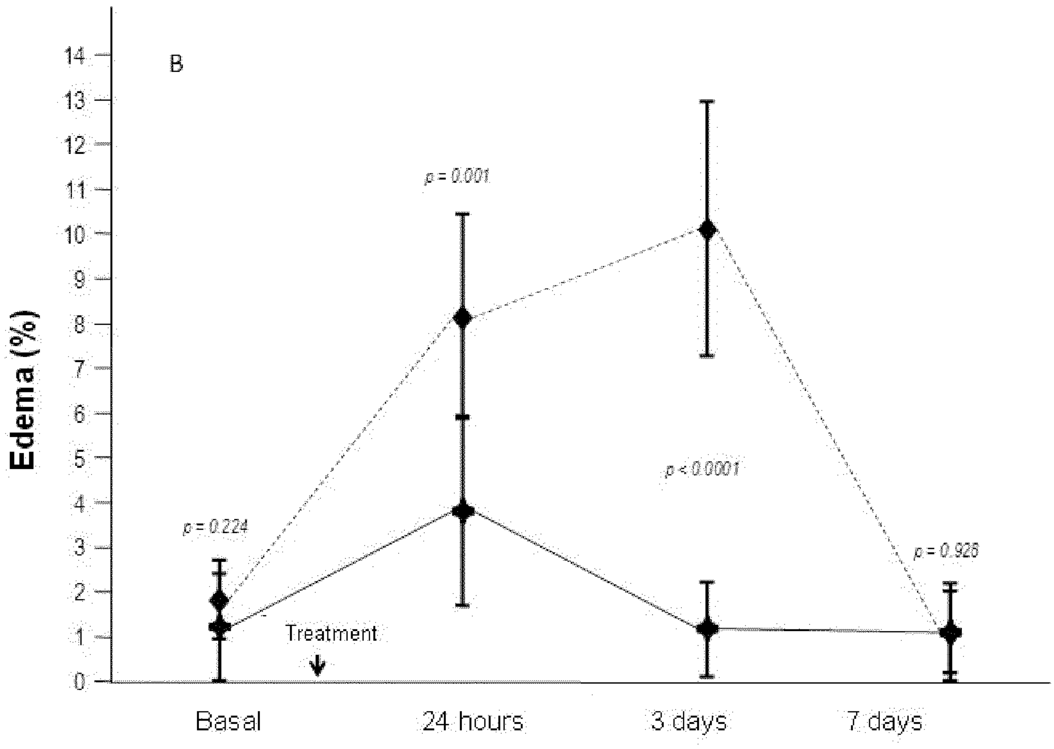
B
Edema (%)
14
13
12
11
10
9
8
7
6
5
4
3
2
1
0
p = 0.001
p < 0.0001
p = 0.224
p = 0.926
Treatment
Basal
24 hours
3 days
7 days

Figure 3b

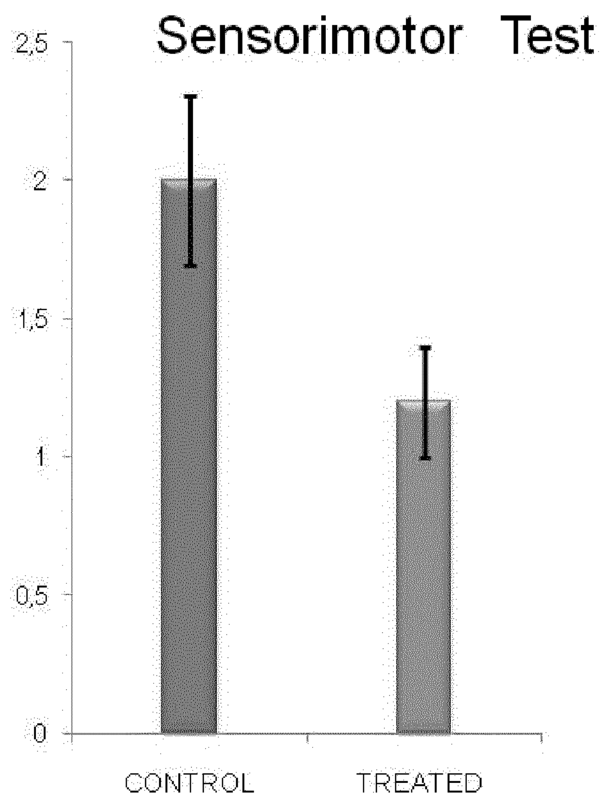


Figure 4

## INTERNATIONAL SEARCH REPORT

International application No.
PCT/ES2011/070344

A. CLASSIFICATION OF SUBJECT MATTER

**See extra sheet**

According to International Patent Classification (IPC) or to both national classification and IPC

B. FIELDS SEARCHED

Minimum documentation searched (classification system followed by classification symbols)
A61K, A61P

Documentation searched other than minimum documentation to the extent that such documents are included in the fields searched

Electronic data base consulted during the international search (name of data base and, where practicable, search terms used)

EPODOC, INVENES, CAS, MEDLINE, BIOSIS, EMBASE, NPL

C. DOCUMENTS CONSIDERED TO BE RELEVANT

| Category* | Citation of document, with indication, where appropriate, of the relevant passages | Relevant to claim No. |
|---|---|---|
| P,X | CAMPOS, FRANCISCO, et al.; Neuroprotection by glutamate oxaloacetate transaminase in ischemic stroke: an experimental study; Journal of Cerebral Blood Flow & Metabolism (2011), 31(6), 1378-1386; ISSN 0271-678X; publicado in line 26.01.2011. | 1-32 |
| X | US 7404951 B2 (TEICHBERG) 29.07.2008, column 1, lines 17-20; column 3, line 49 – column 4, line 34; column 5, lines 17-23; column 14, lines 22-30; column 15, lines 43-37; column 17, lines 58-65; column 18, line 62 – column 19, line 21; example 2. | 1-6, 10-12, 19, 20-22, 32 |

☒ Further documents are listed in the continuation of Box C. ☒ See patent family annex.

* Special categories of cited documents:
"A" document defining the general state of the art which is not considered to be of particular relevance.
"E" earlier document but published on or after the international filing date
"L" document which may throw doubts on priority claim(s) or which is cited to establish the publication date of another citation or other special reason (as specified)
"O" document referring to an oral disclosure use, exhibition, or other means.
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other documents , such combination being obvious to a person skilled in the art
"&" document member of the same patent family

| Date of the actual completion of the international search | Date of mailing of the international search report |
|---|---|
| 29/09/2011 | **(17/10/2011)** |
| Name and mailing address of the ISA/<br><br>OFICINA ESPAÑOLA DE PATENTES Y MARCAS<br>Paseo de la Castellana, 75 - 28071 Madrid (España)<br>Facsimile No.: 91 349 53 04 | Authorized officer<br>N. Vera Gutierrez<br><br>Telephone No. 91 3495544 |

Form PCT/ISA/210 (second sheet) (July 2009)

## INTERNATIONAL SEARCH REPORT

International application No.
PCT/ES2011/070344

C (continuation). DOCUMENTS CONSIDERED TO BE RELEVANT

| Category * | Citation of documents, with indication, where appropriate, of the relevant passages | Relevant to claim No. |
|---|---|---|
| X | US 2009304661 A1 (TEICHBERG ET AL.) 10.12.2009, paragraphs [0001], [0020]-[0023], [0042], [0043], [0074], [0093], [0107], [0180], [0121], [0136], [0152], [0153]. | 1-6, 10-12, 19, 20-22, 32 |

Form PCT/ISA/210 (continuation of second sheet) (July 2009)

**INTERNATIONAL SEARCH REPORT**

International application No.
PCT/ES2011/070344

**Box No. II Observations where certain claims were found unsearchable (Continuation of item 2 of first sheet)**

This international search report has not been established in respect of certain claims under Article 17(2)(a) for the following reasons:

1. ☒ Claims Nos.: **20-32**
because they relate to subject matter not required to be searched by this Authority, namely:

Claims 20-32 relate to a method for treatment of the human or animal body by therapy.
The search was carried out on the basis of the possible effects of the composition.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## INTERNATIONAL SEARCH REPORT

Information on patent family members

International application No.
PCT/ES2011/070344

| Patent document cited in the search report | Publication date | Patent family member(s) | Publication date |
|---|---|---|---|
| US7404951 B | 29.07.2008 | WO2004012762 A | 12.02.2004 |
| | | CA2494348 A | 12.02.2004 |
| | | AU2003247143 A | 23.02.2004 |
| | | AU2003247143 B | 16.07.2009 |
| | | EP1524989 A | 27.04.2005 |
| | | US2006024284 A | 02.02.2006 |
| | | US2008233099 A | 25.09.2008 |
| | | US7767204 B | 03.08.2010 |
| | | US2008233096 A | 25.09.2008 |
| US2009304661 A | 10.12.2009 | WO2007105203 A | 20.09.2007 |
| | | CA2645678 A | 20.09.2007 |
| | | AU2007226134 A | 20.09.2007 |
| | | EP2007206 A | 31.12.2008 |
| | | JP2009530266 A | 27.08.2009 |

Form PCT/ISA/210 (patent family annex) (July 2009)

**INTERNATIONAL SEARCH REPORT**

International application No.
PCT/ES2011/070344

CLASSIFICATION OF SUBJECT MATTER

***A61K31/194*** (2006.01)
***A61K9/08*** (2006.01)
***A61P9/00*** (2006.01)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ADAMS, H.P., JR. et al.** Classification of subtype of acute ischemic stroke. Definitions for use in a multicenter clinical trial. *TOAST. Trial of Org 10172 in Acute Stroke Treatment. Stroke,* 1993, vol. 24 (1), 35-41
- **CASTELLANOS, M. ; T. SOBRINO ; J. CASTILLO.** Evolving paradigms for neuroprotection: molecular identification of ischemic penumbra. *Cerebrovasc Dis,* 2006, vol. 21 (2), 71-9
- **PHILIP, M. et al.** Methodological quality of animal studies of neuroprotective agents currently in phase II/III acute ischemic stroke trials. *Stroke,* 2009, vol. 40 (2), 577-81
- **LIPTON, P.** Ischemic cell death in brain neurons. *Physiol Rev,* 1999, vol. 79 (4), 1431-568
- **CASTILLO, J. et al.** Neuroexcitatory amino acids and their relation to infarct size and neurological deficit in ischemic stroke. *Stroke,* 1996, vol. 27 (6), 1060-5
- **CASTILLO, J. ; A. DAVALOS ; M. NOYA.** Progression of ischaemic stroke and excitotoxic amino acids. *Lancet,* 1997, vol. 349 (9045), 79-83
- **NAGY, D. et al.** Oxaloacetate decreases the infarct size and attenuates the reduction in evoked responses after photothrombotic focal ischaemia in the rat cortex. *Cell Mol Neurobiol,* 2009, vol. 29 (6-7), 827-35
- Dose translation from animal to human studies revisited. *The FASEB Journal,* 2007, vol. 22, 659-661
- **CARMICHAEL, S.T.** Rodent models of focal stroke: size, mechanism, and purpose. *NeuroRx,* 2005, vol. 2 (3), 396-409
- **LONGA, E.Z. et al.** Reversible middle cerebral artery occlusion without craniectomy in rats. *Stroke,* 1989, vol. 20 (1), 84-91
- **WHITE, J.A. ; R.J. HART ; J.C. FRY.** An evaluation of the Waters Pico-Tag system for the amino-acid analysis of food materials. *J Automat Chem,* 1986, vol. 8 (4), 170-7
- **REGLODI, D. ; A. TAMAS ; I. LENGVARI.** Examination of sensorimotor performance following middle cerebral artery occlusion in rats. *Brain Res Bull,* 2003, vol. 59 (6), 459-66